# EUROPEAN PATENT SPECIFICATION

(11) **EP 1 991 138 B1**
(45) Date of publication and mention of the grant of the patent: **16.06.2010**
(21) Application number: 07752060.9
(22) Date of filing: 01.03.2007
(51) Int. Cl.: A61B 17/22

(54) **STENT-CLEANING ASSEMBLY**
ANORDNUNG ZUR STENTREINIGUNG
ASSEMBLAGE DE NETTOYAGE DE STENT

(30) Priority: 08.03.2006 US 780111 P
(43) Date of publication of application: 19.11.2008
(73) Proprietor: Wilson-Cook Medical Inc., Winston-Salem, NC 27105-4191 (US)
(72) Inventor: WALLER, David, F., Winston-Salem, North Carolina 27101 (US)
(74) Representative: Garratt, Peter Douglas
(86) International application number: PCT/US2007/005334
(87) International publication number: WO 2007/103167

(56) References cited:
- US-A- 5 902 263
- US-A- 5 941 869
- US-A- 6 165 209
- US-A1- 2003 109 837

## Description

### CROSS-REFERENCE TO RELATED APPLICATIONS

This application claims priority to U.S. Provisional Application Ser. No. 60/,780,111 , filed March 8, 2006.

### FIELD OF THE INVENTION

The present invention relates generally to medical devices, and more specifically to medical devices configured for use with stents placed within a body lumen or organ of a patient. In particular, the present invention relates to devices and methods for clearing occlusions from stents.

### BACKGROUND

Stents are used to treat occluded vessels, lumens, or organs in various physiological systems of a patient's body. For example, stents are used in the circulatory system for treatment of blood vessel occlusions by introduction of a clear and open passage through the occlusions to unblock, for example, coronary arteries. As another example, stents are used to treat occlusions within the biliary system. Specifically, if a disease condition such as a tumor or an infection-related swelling causes a stenosis or other occlusion of the common bile duct, a stent may be introduced to provide an open, patent passage through the occluded region.

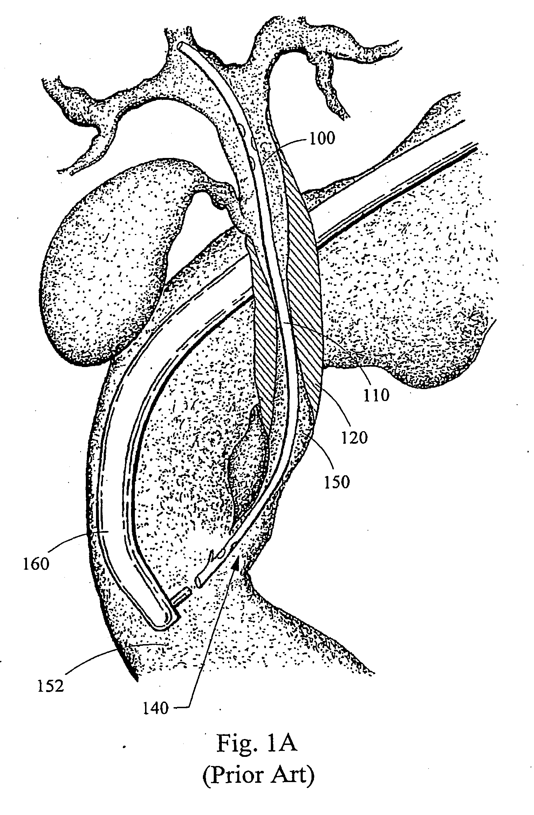

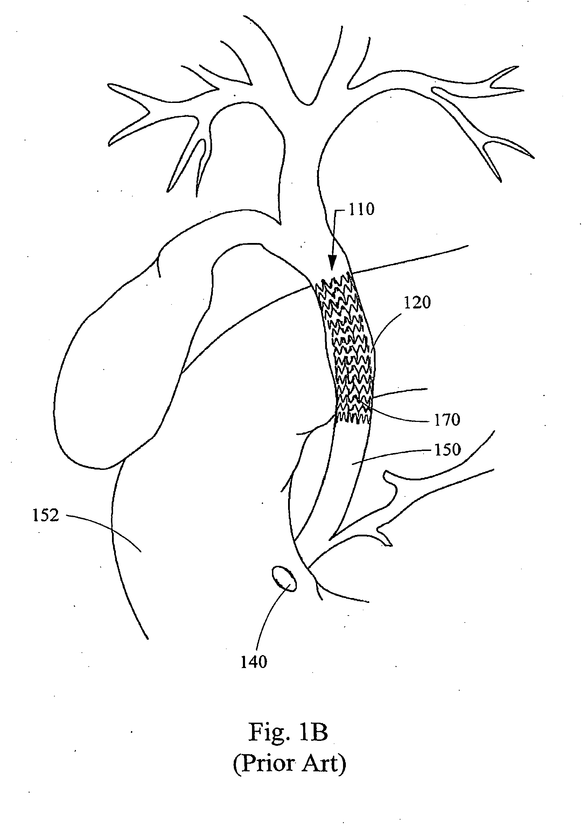

By way of illustration, FIG. 1A shows a plastic biliary stent 100 implanted in the common bile duct 150. The plastic biliary stent 100 provides a patent lumenal passage 110 through a stenosis 120 in the common bile duct 150. FIG. 1A also illustrates an endoscope 160 in the duodenum 152 adjacent the Ampulla of Vater 140, through which a proximal end of the biliary stent 100 extends. The endoscope 160 facilitates the placement and visual assessment of the stent 100 FIG. 1 B depicts an expandable metal biliary stent 170 implanted in the common bile duct 150. Like the plastic stent 100 illustrated in FIG. 1A, the metal biliary stent 170 provides a patent lumenal passage 110 through a stenosis 120 in the common bile duct 150.

Once in place, stents (e.g., biliary stents, coronary stents) may become occluded by deposits from material passing therethrough. For example, a biliary stent may become occluded by deposits of biliary sludge (which commonly includes cholesterol crystals, calcium salts, and mucous) and/or microbiological organisms adhering to the interior diameter of the stent. Several methods are employed to address the problems presented by occlusion of existing stents. Each of the methods typically includes cannulation of the stenosis or other occlusion by at least a wire guide. One method is to inflate a balloon within the occluded region of the stent to compress or dislodge the occluding material and thereby re-establish at least some patency of the stent's lumenal space. Because this method is unlikely to completely remove the occluding material, re-stenosis may occur more rapidly than did the initial occlusion formation (e.g., because the occlusion already has "a foothold" to which more occluding material may be anchored and/or because the full, initial patency of the stent's internal diameter is not reestablished).

Another method is to place a second, smaller stent coaxially within the occluded stent. This method does provide a "clean," patent lumen, but is almost certain to provide a smaller lumenal cross-section in the second, smaller stent than was present in the first stent. As a result, re-stenosis may occur more quickly than did the initial stenosis formation.

Still another method is stent replacement. Removal and replacement of the stent provides a new, clean, open, and patent lumen. However, the procedure is time-consuming and may include increased risks if tissue around the stent has adhered to it (or, in the case of open- sided metal stents, such as expandable stents, surrounding tissue may have invaded the stent itself).

Reference is directed to US 6 165 209 which discloses a stent which is specially adapted for inhibiting restenosis within the stent after implementation in a body passage. The stent may have an internal rail which guides a brush or other stenotic material removal mechanism attached to a catheter. Reference is also directed to US 5 902 263 which discloses apparatus and methods for recannulising stented regions within the vasculature which have become restenosed. A compliant shearing body such as a brush is displaced within the stented region. For example, the shearing body may be mounted on a shaft which can be rotated by a motor. Finally, reference is directed to US 5 941 869 which disclosed apparatus and methods for treating in-stent restenosis. A stenotic material removal mechanism may take the form of a cutter and a balloon may be provided to control the depth of cut.

As a result, there still exists a need for an efficient, effective method and/or device for treatment of stenosis or other occlusion of the lumenal space of stents.

### BRIEF SUMMARY

The present invention is defined in the appended claims. The present invention presents devices and methods for treating stenoses or other occlusions that may occur in stents that are deployed within a body lumen of a patient. In one aspect, embodiments of the present invention may include a stent-cleaning assembly that preferably is actuatable through an endoscope. The stent cleaning assembly may have a catheter component, including an engagement member configured to hold the catheter component generally stationary relative to a stent, and a deployable cleaning member, including a frictional cleaning surface such as a brush, that may be deployed from the catheter to contact material in the stent.

### BRIEF DESCRIPTION OF THE DRAWINGS

FIG. 1A is a first simplified illustration of a portion of the biliary system and shows a plastic biliary stent and an endoscope;

FIG. 1B is a second simplified illustration of a portion of the biliary system and shows an expandable metal biliary stent.

FIG. 2 depicts a side view of a first embodiment of a stent- cleaning brush assembly;

FIG. 3 illustrates the brush assembly of FIG. 2 engaged with a stent;

FIG. 4 illustrates a side view of a stent-cleaning brush assembly;

FIGS. 5A-5I schematically depict side views of exemplary bristle configurations for use with the present invention;

FIGS. 6A-6E schematically depict transverse cross-sectional views of exemplary bristle configurations for use with the present invention;

FIG. 7 illustrates a longitudinal cross-section of the distal portion of a third embodiment of a stent-cleaning brush;

FIG. 8 shows another stent cleaning device embodiment; and

FIG. 9 depicts a stent cleaning assembly embodiment configured with an expandable engagement member.

### DETAILED DESCRIPTION

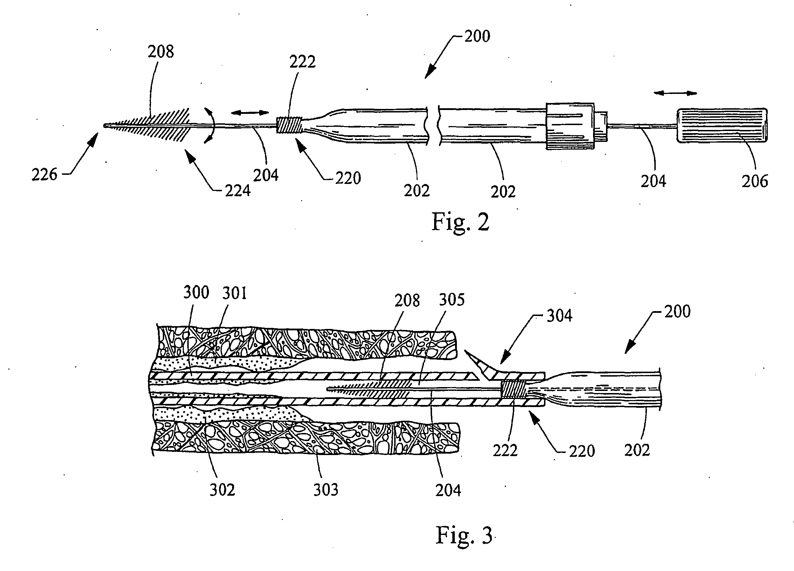

FIG. 2 illustrates an external side view of one embodiment of a stent-cleaning brush assembly 200. The assembly 200 includes an elongate catheter body 202, with a flexible, axially actuatable shaft 204 extending therethrough. The shaft 204 includes a control handle 206 at its proximal end and a cleaning member embodied as a bristled cleaning brush 208 near its distal end. A cleaning member may alternatively be embodied as a non-brush structure, such as - for example - an abrasive foam member (e.g., a closed cell sponge), a filamentous pad (e.g., a scrubbing pad), or another structure with an appropriately configured cleaning surface, all within the scope of the present invention. A distal end portion 220 of the catheter body 202 preferably will be tapered to a smaller outer diameter than the rest of the catheter body 202, or may otherwise be dimensioned to effectively engage a stent, or a location near a stent enabling access of the cleaning member to the stent. In the illustrated embodiment, the distal catheter end portion 220 includes an engagement member embodied as a threaded surface having generally helically disposed external threads 222. An engagement member will generally be configured to engage a stent in a manner limiting movement of the stent relative to the cleaning assembly. In preferred embodiments having a threaded engagement member, the sizes of the distal end portion 220 and of the external threads 222 will be such that they will fit engagingly into a proximal end of a stent to be cleaned.

As is shown in the embodiment illustrated in FIG. 2, the bristles of the cleaning brush 208 may be configured such that the bristles taper from a longer bristle length near the proximal brush portion 224 to a shorter bristle length near the distal brush portion 220. The brush 208 and/or the shaft 204 may include indicia for visualization (e.g., colored or fluorescent indicia for optical visualization or radio-opaque markers for fluoroscopic visualization). Also, as is described in greater detail below, the cleaning brush can comprise any number of shape configurations and/or may include a variety of types and textures of bristles (with the term "bristles" encompassing other structures such as rounded projections or other shaped components of the cleaning member).

In certain preferred embodiments, the cleaning assembly 200 may be configured for introduction into and operation within a patient body through an endoscope such as a duodenoscope. In other preferred embodiments, the assembly may be of a different scale and configured for introduction into a blood vessel of a patient. For example the assembly may be configured for percutaneous introduction through the femoral artery and being directed to the coronary arteries of a patient.

In another embodiment, the catheter body 202 may be configured to attach to a vacuum device. Such an embodiment preferably will include a catheter body 202 that is able to withstand a vacuum, and may be used with a vacuum device configured to suctionally remove material being cleared from the stent.

FIG. 3 depicts a distal portion of the brush assembly 200 engaged with a stent 300 in need of cleaning. The stent 300 is disposed in a stenotic region 301 of a vessel 303 and has material 302 occluding the stent lumen 305 (which corresponds to an internal diameter of the stent 300). The threads 222 of the catheter body 202 enable the distal end of the catheter body 202 to engage with the proximal end of the stent 300. By holding the proximal end of the catheter body 202 stationary, the user can stabilize and prevent movement of the stent 300 during the cleaning operation. This preferably decreases the likelihood of the stent 300 from being dislodged or harming the surrounding tissue, and further permits a back and forth movement of the brush 208 during a cleaning operation without significantly moving the stent 300. In particular, the internal diameter of the stent 300 is engaged by the catheter threads 222 as the tapered distal catheter end portion 220 is directed into a proximal end portion 304 of the stent 300. One example of a catheter-type device that may be useful for threadedly engaging a stent is a Soehendra(R) Stent Retriever (Cook Endoscopy G21934 et al., Winston-Salem, NC) (See also U.S. Pat. Nos. 5,334,208 and 5,643,277.

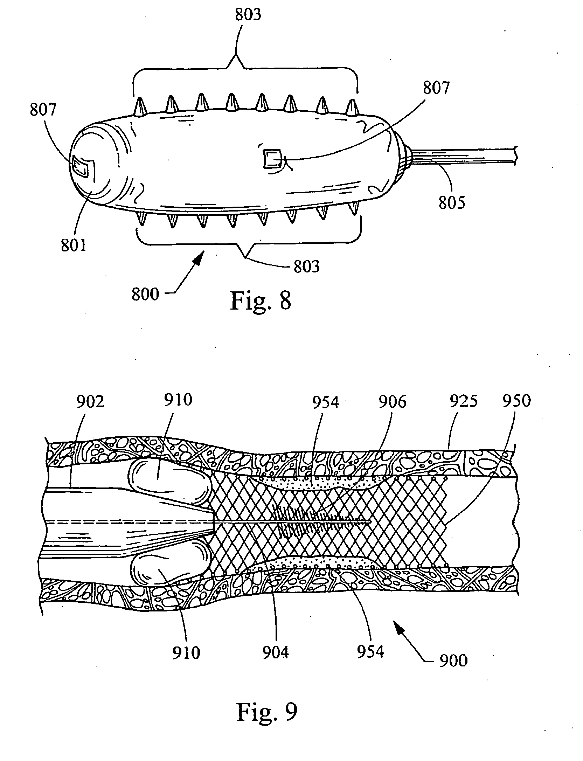

In one application of the assembly 200, the threads 222 may be rotated such that they engage the internal diameter of the proximal end portion 304 of the stent 300. Preferably the size and the composition of the threads 222 will be configured such that there is little or no permanent change or damage to the lumenal surface of the stent 300. Those of skill in the art will appreciate that a friction-fit-balloon (see FIG. 9), a deployable set of prongs, or other connecting means may be used to engage the brush assembly to a stent in need of cleaning. Moreover, stents may be provided that include, for example, threads, bayonet-mating structure, embedded magnets, or other connecting structure configured to engage with an appropriately configured distal catheter portion of the present invention. However, the threaded configuration of the illustrated embodiment of FIG. 2 includes an advantage that the stent need not be provided with a complementary engaging structure.

After the brush assembly 200 is engaged with the proximal end portion 304 of the stent 300, a user may actuate the brush 208 by moving the handle 206 distally relative to the catheter body 202 to extend the brush 208 out of the distal end portion 220 of the catheter body 202 and then by moving the handle 206 such that the brush 208 contacts and dislodges occlusive material 302. Axial back-and-forth movements of the handle 206 (see FIG. 2), as well as rotational movements, are translated through the shaft 204 to axial and rotational movements of the brush 208 within the stent 300. This causes the material 302 to be removed from the interior surface of the stent 300 and opens/widens the passageway therethrough. In some embodiments, a motorized mechanism may be provided for rotating, reciprocating, or otherwise moving the cleaning member.

Preferably, the greatest outer diameter of the body of brush bristles 208 will be equal to or slightly greater than the internal diameter of the stent 300, so that adequate friction will be generated to dislodge some, most, or all of the occluding material 302. Most preferably, the composition of the brush bristles 208 is such that occluding material may be removed thereby with little or no abrasion of the interior diameter of the stent being cleaned. Appropriate materials for use as brush bristles may include, for example, nylon, polyethylene, other rigid non-toxic plastics, stainless steel wire, or other non-corrosive, non-toxic metals. The body of bristles of a single brush may include several different materials (e.g., a mixture of stiffer or more abrasive bristles with more flexible or less abrasive bristles). Preferred qualities of materials for use in the core are similar to the qualities preferred for wire guides. Specifically, the core material preferably allows accurately controlled rotary and longitudinal movement of the distal/brush end by movement of the proximal/handle end of the device. Preferred core materials include nitinol and stainless steel.

After the brush 208 has been actuated to clean out and re-open the occluded portion of the stent 300, the brush 208 will be withdrawn into the catheter body 202 by drawing the handle 206 distally. Then, the catheter body 202 is rotated to disengage the threads 222 from the internal diameter of the stent 300. Preferably, this operation is accomplished with little or no significant movement of the stent 300.

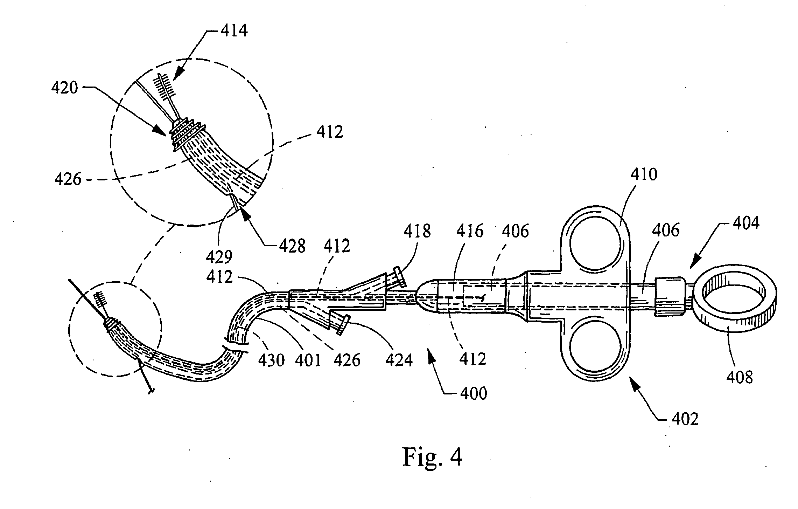

FIG. 4 illustrates an alternative embodiment of a stent-cleaning brush assembly 400, with the distal end portion magnified to show certain details. The stent cleaning brush assembly includes an elongate tubular catheter body 401. A three-ring handle 402 forms the proximal end portion of the assembly 400. The three-ring handle 402 includes a single-ring/thumb-ring element 404, which preferably is configured for both axial sliding movement along and rotation around its central longitudinal axis, relative to a two-ring element 410. The single-ring element 404 includes a shaft portion 406 and a ring portion 408. Near the distal end of the shaft portion 406, the single-ring element 404 is attached to the proximal portion of an elongate flexible shaft 412, which extends through a first lumen 430 in the catheter body 401 and terminates distally in a stent-cleaning member embodied as a brush 414. A wire guide lumen 426 also extends through the length of the catheter body 401. Adjacent the distal end of the catheter body 401, a rapid-exchange side port 428 is open to the wire guide lumen 426. A wire guide 429 preferably is used in guiding the assembly 400 to a target location. The wire guide 429 is shown in the magnified view of the end portion, and is directed through the rapid-exchange side port 428.

The proximal portion of two-ring element 410 includes a central cavity 416, in which the shaft portion 406 of the single-ring element 404 is movably disposed. The distal portion of the two-ring element 410 includes an optional side port 418 open to the first lumen 430. The side port 418 preferably is configured for use in introducing, for example, a contrast fluid, a solution for flushing the catheter 401, or a solution (such as an enzyme solution or other type of solution) for helping to break down a stenotic build-up in a stent. The side port 418 and first lumen 430 may also be used for applying a vacuum to remove (by suction) material that is dislodged by the brush 414. The distal portion of the two-ring element 410 also includes a wire guide port 424 that provides access to the wire guide lumen 426. Together with the rapid-exchange side port 428 the wire guide port 424 provides the brush assembly 400 with "convertible catheter" functionality, wherein it may be used in a long-wire or a short wire/rapid-exchange configuration. The distal portion of the two-ring element 410 is attached to the catheter body 401. The distal end of the catheter body 401 includes a threaded surface 420, preferably configured for engagement with a stent.

An operation of the assembly 400 is also described with reference to FIG. 4. The assembly 400 may be directed over a wire guide 429 to a location adjacent a stent (not shown) in need of cleaning. The threaded surface 420 may then be engaged to the stent by rotating the two-ring element 410, thereby transmitting rotational movement through the catheter 401 in a manner allowing an engagement surface including threads 420 to advance into the stent, securing the catheter 401 to the stent. The flexible shaft 412 may be advanced distally by actuation of the single-ring element 404, and the brush body 414 employed to engage a stenosis in the stent (for example, in a manner similar to that described above with reference to FIG. 3). Axial or twisting movements of the single-ring element 402 preferably are translated to axial or twisting movements of the brush body 414 in a manner useful for clearing stenotic material from the stent.

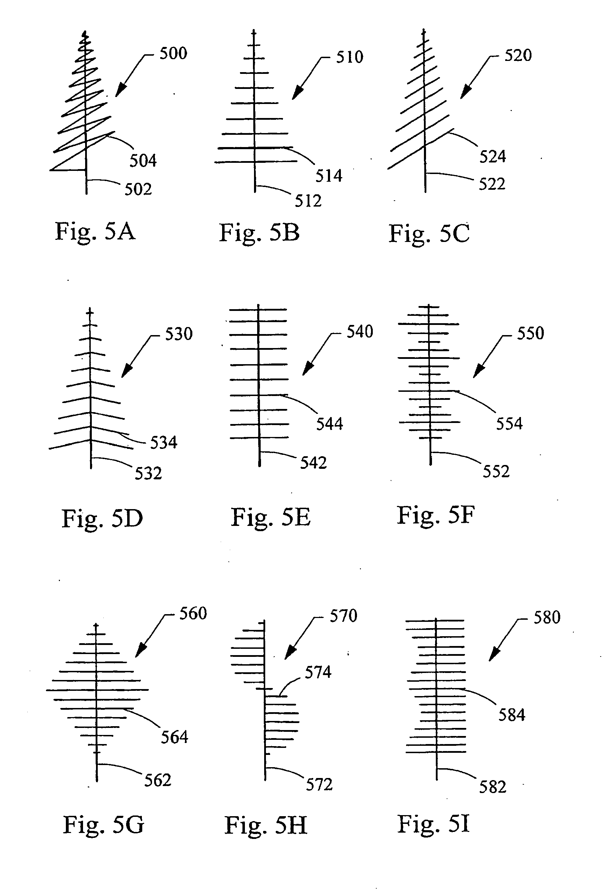

For a cleaning member of the present invention, the bristles or other cleaning member elements of a brush portion (in, for example, a bristle body) may be configured in many different ways. For example, the bristles may be constructed of materials and/or configured in patterns to collect the material being removed from the stent, or just to dislodge the material. FIGS. 5A-5I are side-view profile illustrations that diagrammatically depict bristle body profile embodiments. It should be appreciated that combinations or variations of these examples, as well as other brush configurations, are within the scope of the present invention. FIG. 5A illustrates a brush 500 with a central shaft 502 and bristles 504 attached to the shaft 502 in a tapered tight-spiral configuration. FIG. 5B illustrates a brush 510 with a central shaft 512 and bristles 514 attached to the shaft 512 in a tapered configuration, wherein the bristles 514 are in tiers that are generally perpendicular to the shaft 512. FIG. 5C illustrates a brush 520 with a central shaft 522 and bristles 524 attached to the shaft 522 in a tapered configuration, wherein the bristles 524 are in tiers that are generally oriented at an angle relative to the longitudinal axis of the shaft 522.

FIG. 5D illustrates a brush 530 with a central shaft 532 and bristles 534 attached to the shaft 532 in a tapered configuration, wherein the bristles 534 are in tiers that are each generally oriented in a conical pattern, the apex of which is near the longitudinal axis of the shaft 532. In this and other angled bristle configurations, the bristles may be angled toward or away from the distal end. FIG. 5E illustrates a brush 540 with a central shaft 542 and bristles 544 attached to the shaft 542 in an un-tapered configuration, wherein the bristles 544 are in tiers of a generally consistent circumference. FIG. 5F illustrates a brush 550 with a central shaft 552 and bristles 554 attached to the shaft 552 in an un-tapered configuration, wherein the bristles 554 are in tiers and the tiers have different circumferences alternating between larger and smaller circumferences.

FIG. 5G illustrates a brush 560 with a central shaft 562 and bristles 564 attached to the shaft 562 in a "dual-tapered" configuration wherein the bristles 564 are in tiers and the tiers have different circumferences. Specifically, the tiers of bristles 564 have a larger circumference near the middle of the brush 560 and smaller circumference near the ends of the brush 560. In this and other example brush embodiments, the same or a similar outer contour of the brush bristles may be accomplished with bristles arranged in spiral, linear or other configurations rather than tiered configurations. FIG. 5H illustrates a brush 570 with a central shaft 572 and bristles 574 attached to the shaft 572 in an un-tapered loose-spiral configuration. FIG. 5I illustrates a brush 580 with a central shaft 582 and bristles 584 arranged in generally off-center circular tiers on the shaft 582.

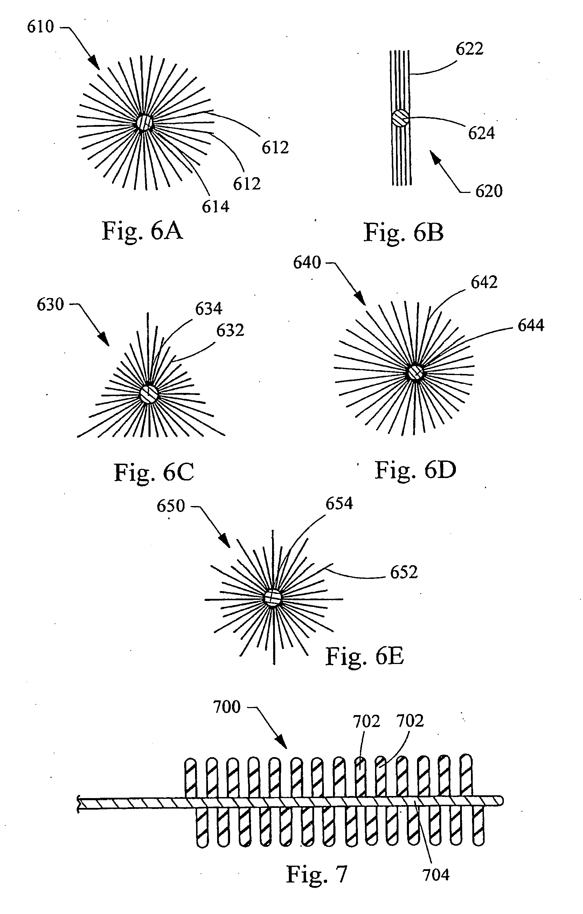

In addition to and/or in conjunction with the bristle body embodiments shown in FIGS. 5A-5I, embodiments of brushes of the present invention may include a number of different bristle configurations as viewed in transverse cross-section. These configurations may be used in conjunction with bristle body embodiments described with reference to FIGS. 5A-5I. Some exemplary bristle body embodiments are illustrated in the transverse cross-sectional views of FIGS. 6A-6E, but it should be appreciated that other configurations may be practiced within the scope of the present invention. FIG. 6A depicts a cross-sectional view of a center-round bristle configuration of a brush 610 wherein bristles 612 are arranged to have a generally circular outer profile centered around a shaft 614. FIG. 6B depicts a cross-sectional view of a center-linear bristle configuration of a brush 620 wherein bristles 622 are arranged generally parallel such that they present a generally linear profile centered on a shaft 624. FIG. 6C depicts a cross-sectional view of an off-center-triangle bristle configuration of a brush 630 wherein bristles 632 are arranged to have a generally triangular outer profile that is nearly centered around a shaft 634. FIG. 6D depicts a cross-sectional view of an off-center-round bristle configuration of a brush 640 wherein bristles 642 are arranged to have a generally circular outer profile asymmetrically oriented around a shaft 644. FIG. 6E depicts a cross-sectional view of a bristle configuration of a brush 650 wherein bristles 652 of different lengths are centered around a shaft 654. It should be appreciated that other geometric shapes of the outer profile of the bristles are within the scope of the present invention.

FIG. 7 shows an embodiment of a stent-cleaning device as a brush 700 of the present invention wherein the brush 700 includes a plurality of finger-like projections 702 disposed on a central shaft 704. In alternative embodiments, the finger-like projections 702 may be configured on the shaft 704 as described above for brush bristles or in other configurations (e.g., differing lengths, outer profiles, patterns of placement on the shaft). The finger-like projections 702 preferably are made of a flexible material such as, for example, latex, PTFE, or silicon, but may be of limited flexibility, made of an abrasive material (e.g., an alloy or coarse-surfaced material), and/or treated to have an abrasive surface.

FIG. 8 illustrates another stent-cleaning device embodiment of the present invention as a cleaning tip 800, which includes an expandable cleaning member, embodied as a balloon 801. The balloon 801 preferably comprises a non-compliant body, the maximum expanded outer diameter of which is configured to be at least slightly less than the internal diameter of a stent to be cleaned. The balloon 801 may include a series of protrusions 803 along its surface, which are configured to provide abrasiveness sufficient to remove occluding material from within a stent. Alternatively, the external balloon surface may include an abrasive material such as embedded silicon dioxide.

In one embodiment, the balloon 801 may be disposed adjacent the distal end of a torqueable catheter body 805. In an application of the expandable member cleaning tip 800, the cleaning tip 800 may be mounted adjacent the distal end of a catheter body 805 and directed through an outer catheter (not shown) to the interior of a stent (not shown) having occluding material therein to be removed. The outer catheter preferably is engaged to the stent, as described above with reference to other embodiments (e.g., by a threaded engagement or other engagement means). A set of radio-opaque markers 807 on the catheter body 805 and/or balloon 801 may be provided for fluoroscopic visualization of the assembly 800. The balloon 801 may be inflated in the stent, thereby exerting radial force against the occluding material, and then rotated and/or moved axially such that the protrusions 803 dislodge the occluding material from the stent. In an alternative embodiment, the expandable member may be an expandable basket, comprising an abrasive material or construction that is configured to dislodge occluding material from a stent.

Other embodiments are contemplated within the scope of the present invention including, for example, (1) a cleaning tip comprising a helically-threaded surface that can auger through occluding material as well as being moved axially to displace it; (2) a cleaning tip comprising a serrated polymer surface; or (3) a cleaning tip comprising a corkscrew shape wherein the corkscrew body optionally includes one or more abrasive surfaces.

FIG. 9 is a cut-away view of a vessel 925 in a patient body. The vessel 952 has an expandable stent 950 disposed therein. The stent 950 has deposited material 954 occluding it. FIG. 9 also shows another embodiment of a stent-cleaning assembly 900 including an outer catheter 902 and an inner cleaning member 904 extending through the outer catheter 902. A serrated or otherwise irregular cleaning tip 906 is disposed adjacent the distal end of the inner cleaning member 904. An expandable engagement member, embodied as a balloon 910 is disposed adjacent the distal end of the outer catheter 902. As shown in FIG. 9, the balloon 910 is expanded within a proximal portion of the stent 950 to engage the assembly 900 to the stent 950. The balloon 910 may be constructed of a compliant or non-compliant material and preferably includes an abrasive or adhesive surface that enhances its ability to remain engaged with the stent 950 and/or with the wall of the vessel 952 adjacent the stent 950 during a cleaning operation.

It is intended that the foregoing detailed description be regarded as illustrative rather than limiting, and that it be understood that the following claims, including all equivalents, are intended to define scope of this invention.

## Claims

1. A device (200) configured for cleaning a stent, comprising:
an elongate catheter body (202),
an engagement member disposed adjacent a distal end (220) of the catheter body, the engagement member comprising a threaded exterior surface (222) configured to threadingly engage an interior diameter of a stent;
an elongate shaft (204) having a proximal end and a distal end and disposed movably extending through the catheter body; and
a cleaning member (208) mounted on the elongate shaft and disposed adjacent the distal end of the elongate shaft.

2. The device of claim 1, further comprising a handle (206) disposed on the proximal end of the shaft

3. The device of claim 2, wherein the handle assembly comprises a two-part handle, comprising:
a first handle structure connected to the elongate catheter body;
a second handle structure mounted to the first handle structure in a manner allowing proximal and distal sliding movement of the second handle structure relative to the first handle structure;
the second handle structure comprising a proximal attachment to the elongate shaft; and
wherein movement of the second handle structure relative to the first handle structure causes corresponding movement of the elongate shaft relative to the elongate catheter body, and preferably
wherein an axial movement of the second handle structure translates to axial movement of the elongate shaft.

4. The device of claim 1, wherein the cleaning member comprises a brush.

5. The device of claim 4, wherein the brush comprises a generally conical body of bristles that tapers from a broader portion proximally to a narrower portion distally, and preferably wherein the body of bristles comprises longitudinal rows of bristles.

6. The device of claim 4, wherein the brush comprises a body of bristles, the body having a generally triangular transverse cross-section.

7. The device of claim 4, wherein the brush comprises first bristles having a first abrasiveness or flexibility, and second bristles having a second abrasiveness or flexibility.

8. The device of claim 4 comprising at least one of tier of bristles disposed at an acute angle relative to a longitudinal axis of the elongate shaft.

9. The device of claim 4, wherein the brush includes a plurality of bristles, the ends of which are angled in a generally proximal direction.

10. The device of claim 1, wherein the cleaning member includes a plurality of flexible projections extending generally laterally from the elongate shaft.

## Patentansprüche

1. Vorichtung (200) zur Stentreinigung, die Folgendes umfasst: einen länglichen Katheterkörper (202), ein Eingriffselement neben einem distalen Ende (220) des Katheterkörpers, wobei das Eingriffselement eine mit Gewinde versehene Außenseite (222) umfasst, die mit einem Innendurchmesser eines Stents in Gewindeeingriff kommen kann; einen länglichen Schaft (204) mit einem proximalen Ende und einem distalen Ende, der durch den Katheterkörper beweglich angeordnet ist; und ein Reinigungselement (208), das auf dem länglichen Schaft befestigt ist und neben dem distalen Ende des länglichen Schafts angeordnet ist.

2. Vorrichtung nach Anspruch 1, die ferner einen auf dem proximalen Ende des Schafts angeordneten Handgriff (206) umfasst.

3. Vorrichtung nach Anspruch 2, worin die Handgriffanordnung einen zweiteiligen Handgriff umfasst, der Folgendes umfasst: eine erste Handgriffkonstruktion, die mit dem länglichen Katheterkörper verbunden ist; eine zweite Handgriffkonstruktion, die so auf der ersten Handgriffkonstruktion befestigt ist, dass eine proximale und distale Schiebebewegung der zweiten Handgriffkonstruktion relativ zu ersten Handgriffkonstruktion möglich ist; wobei die zweite Handgriffkonstruktion eine proximale Befestigung am länglichen Schaft umfasst; und worin die Bewegung der zweiten Handgriffkonstruktion relativ zur ersten Handgriffkonstruktion eine entsprechende Bewegung des länglichen Schafts relativ zum länglichen Katheterkörper hervorruft, und worin vorzugsweise eine axiale Bewegung der zweiten Handgriffkonstruktion in eine axiale Bewegung des länglichen Schafts übersetzt wird.

4. Vorrichtung nach Anspruch 1, worin das Reinigungselement eine Bürse umfasst.

5. Vorrichtung nach Anspruch 4, worin die Bürste einen allgemein konischen Borstenkörper umfasst, der sich von einem breiteren Abschnitt proximal zu einem schmaleren Abschnitt distal verjüngt, und worin der Borstenkörper vorzugsweise Längsreihen von Borsten umfasst.

6. Vorrichtung nach Anspruch 4, worin die Bürste einen Borstenkörper umfasst, wobei der Körper einen allgemein dreieckigen Querschnitt aufweist.

7. Vorrichtung nach Anspruch 4, worin die Bürste erste Borsten mit einer ersten Abrasivität oder Flexibilität und die zweiten Borsten eine zweite Abrasivität oder Flexibilität aufweisen.

8. Vorrichtung nach Anspruch 4, die zumindest eine Borstenreihe umfasst, die im spitzen Winkel relativ zu einer Längsachse des länglichen Schafts angeordnet ist.

9. Vorrichtung nach Anspruch 4, worin die Bürste eine Vielzahl von Bürsten aufweist, deren Enden in einer allgemein proximalen Richtung abgewinkelt sind.

10. Vorrichtung nach Anspruch 1, worin das Reinigungselement eine Vielzahl von flexiblen Vorsprüngen aufweist, die sich allgemein seitlich vom länglichen Schaft erstrecken.

## Revendications

1. Dispositif (200) conçu pour nettoyer un tuteur, comportant :
un corps de cathéter allongé (202) ;
un organe d'engrènement disposé à côté d'une extrémité distale (220) du corps de cathéter, l'organe d'engrènement comportant une surface externe filetée (222) conçue pour s'engrener de manière filetée avec le diamètre interne d'un tuteur ;
une tige allongée (204) présentant une extrémité proximale et une extrémité distale et disposée de manière mobile à travers le corps de cathéter ; et
un organe de nettoyage (208) monté sur la tige allongée et disposé à côté de l'extrémité distale de la tige allongée.

2. Dispositif selon la revendication 1, comportant en outre un manche (206) disposé sur l'extrémité proximale de la tige.

3. Dispositif selon la revendication 2, dans lequel l'assemblage de manche comporte un manche en deux parties, comportant :
une première structure de manche reliée au corps de cathéter allongé ;
une deuxième structure de manche montée sur la première structure de manche de façon à permettre un déplacement coulissant proximal et distal de la deuxième structure de manche par rapport à la première structure de manche ;
la deuxième structure de manche comportant une fixation proximale sur la tige allongée ; et
dans lequel le déplacement de la deuxième structure de manche par rapport à la première structure de manche entraîne un déplacement correspondant de la tige allongée par rapport au corps de cathéter allongé, et de préférence
dans lequel un déplacement axial de la deuxième structure de manche se transforme en un mouvement axial de la tige allongée.

4. Dispositif selon la revendication 1, dans lequel l'organe de nettoyage comporte une brosse.

5. Dispositif selon la revendication 4, dans lequel la brosse comporte un corps de poils de brosserie en général conique qui diminue graduellement d'une portion plus large proximalement à une portion plus étroite distalement, et de préférence dans lequel le corps de poils de brosserie comporte des rangées longitudinales de poils de brosserie.

6. Dispositif selon la revendication 4, dans lequel la brosse comporte un corps de poils de brosserie, le corps ayant une section transversale en général triangulaire.

7. Dispositif selon la revendication 4, dans lequel la brosse comporte un premier ensemble de poils de brosserie ayant une première propriété d'abrasivité ou de flexibilité, et un deuxième ensemble de poils de brosserie ayant une deuxième propriété d'abrasivité ou de flexibilité.

8. Dispositif selon la revendication 4 comportant au moins un niveau de poils de brosserie parmi plusieurs qui est disposé de façon à former un angle aigu par rapport à un axe longitudinal de la tige allongée.

9. Dispositif selon la revendication 4, dans lequel la brosse comprend une pluralité de poils de brosserie, dont les extrémités sont disposées en angle dans une direction en général proximale.

10. Dispositif selon la revendication 1, dans lequel l'organe de nettoyage comprend une pluralité de saillies flexibles s'étendant en général latéralement depuis la tige allongée.
